# EUROPÄISCHE PATENTSCHRIFT

(11) **EP 0 505 856 B1**
(45) Veröffentlichungstag und Bekanntmachung des Hinweises auf die Patenterteilung: **06.12.1995**
(21) Anmeldenummer: 92104391.5
(22) Anmeldetag: 13.03.1992
(51) Int. Cl.: A61N 1/39

(54) **Implantierbare Defibrillatoranordnung**
Implantable defibrillator device
Défibrillateur cardiaque implantable

(30) Priorität: 28.03.1991 DE 4110404
(43) Veröffentlichungstag der Anmeldung: 30.09.1992
(73) Patentinhaber: Pacesetter AB, 171 95 Solna (SE)
(72) Erfinder: Andersen, Hans, S-162 35 Vällingby (SE); Hirschberg, Jakub, S-183 44 Täby (SE)
(74) Vertreter: Lettström, Richard Wilhelm

(56) Entgegenhaltungen:
- US-A- 4 834 100

## Beschreibung

Die Erfindung betrifft eine implantierbare Defibrillatoranordnung mit einem in einem Kapselgehäuse angeordneten Kondensator, der über eine steuerbare Schalteranordnung zum Aufladen mit einer spannungsquelle und zur Abgabe eines elektrischen Defibrillationsimpulses über eine Induktivität und Elektrodenzuleitungen mit im Bereich des Herzens angeordneten Elektroden verbindbar ist.

Eine derartige, aus der US-A 4 834 100 bekannte Defibrillatoranordnung weist ein implantierbares Kapselgehäuse auf, innerhalb dessen ein Kondensator mittels einer steuerbaren Schalteranordnung entweder an eine Spannungsquelle oder in Reihenschaltung mit einer Induktivität an zwei Elektrodenanschlüsse schaltbar ist, an denen außerhalb des Kapselgehäuses Elektrodenzuleitungen für am oder im Herzen eines Patienten plazierte Elektroden anschließbar sind. Solange der Kondensator mit der Spannungsquelle verbunden ist, wird er auf eine vorgegebene Ladespannung aufgeladen. Zur Abgabe eines elektrischen Defibrillationsimpulses an das Herz wird der Kondensator mittels der Schalteranordnung kurzzeitig über die Induktivität mit den Elektroden verbunden, wobei ein biphasischer Stromimpuls in Form einer stark gedämpften Schwingung erzeugt wird. Eine derartige Impulsform hat sich in Bezug auf die zur Defibrillation benötigte Energie als besonders effektiv erwiesen. Die Form der Schwingung bestimmt sich aus den Werten für die Kondensatorkapazität, die Induktivität und den elektrischen Widerstand der Herzgewebes zwischen den Elektroden, wobei letzterer in erheblichem Maße von den jeweils verwendeten Elektroden, deren Form und deren Anordnung abhängig ist. Der von der bekannten Defibrillatoranordnung abgegebene Defibrillationsimpuls kann daher erheblich von einer als optimal angesehenen Form abweichen, je nachdem, welche Elektroden in welcher Anordnung verwendet werden.

Der Erfindung liegt daher die Aufgabe zugrunde, eine Defibrillatoranordnung anzugeben, die auf einfache Weise eine Optimierung des abzugebenden Defibrillationsimpulses an unterschiedliche Elektroden und Elektrodenanordnungen ermöglicht.

Gemäß der Erfindung wird die Aufgabe dadurch gelöst, daß bei der Defibrillatoranordnung der eingangs angegebenen Art die Induktivität in einem zweiten, separaten Gehäuse angeordnet ist, das mit Anschlüssen zum Einfügen der Induktivität in den Verlauf einer der Elektrodenzuleitungen versehen ist. Dadurch ist die Möglichkeit gegeben, in Abhängigkeit von den für den Patienten vorgesehenen Elektroden und deren Anordnung am Herzen, die für einen optimalen Defibrillationsimpuls passende Induktivität auszuwählen. Eine derartige Optimierung ist auch bei einer bereits implantierten Defibrillatoranordnung möglich, ohne daß die ganze Anordnung explantiert werden muß, weil lediglich ein Austausch des separaten Kapselgehäuses mit der Induktivität erforderlich ist. Dabei ist insbesondere von Vorteil, daß das separate Gehäuse auf Grund dessen, daß es lediglich die Induktivität beinhaltet, besonders geringe Außenmaße aufweist und daher an für einen späteren Austausch besonders günstigen Stellen im Körper des Patienten implantiert werden kann. Schließlich ist als Vorteil anzusehen, daß bereits vorhandene bzw. bereits implantierte Defibrillatoren, die lediglich zur Abgabe von monophasischen Impulsen ausgebildet sind, auf einfachste Weise zu einer Anordnung weitergebildet werden können, die biphasische Defibrillationsimpulse erzeugt.

Die Möglichkeiten zur Optimierung des abzugebenden Defibrillationsimpulses in Bezug auf unterschiedliche Elektroden und Elektrodenanordnungen lassen sich in vorteilhafter Weise weiter verbessern, indem in dem zweiten, separaten Gehäuse zusätzlich zu der Induktivität zumindest ein weiteres passives elektrisches Bauelement zwischen den Anschlüssen angeordnet ist. Dabei kann es sich um einen Kondensator oder um einen Widerstand handeln, der entweder in Reihe mit der Induktivität oder parallel zu dieser liegt.

Entsprechend einer vorteilhaften Weiterbildung der erfindunggemäßen Defibrillatoranordnung ist vorgesehen, daß das separate Gehäuse zumindest einen zusätzlichen Anschluß für eine weitere Elektrode aufweist und zumindest ein zusätzliches passives elektrisches Bauelement enhält, welches mit der Induktivität zu einer Stromteilerschaltung angeordnet ist, die den von dem Kondensator abgegebenen Defibrillationsimpuls in unterschiedliche Teilströme für die Elektroden aufteilt. Auf diese Weise wird erreicht, daß sich bei der Abgabe des Defibrillationsimpulses die elektrische Stromdichte im Herzmuskel entsprechend der Anordnung der Elektroden verteilt und dabei vorzugsweise die dicksten Zonen des Herzmuskels, welche den Hauptteil der Herzmuskelmasse bilden, durchdringt, um eine sichere Defibrillation zu erzielen. Auch in diesem Falle können bereits vorhandene oder bereits implantierte Defibrillatoren, die lediglich zum Anschluß von zwei Elektroden ausgebildet sind, entsprechend nachgerüstet werden.

Da, wie bereits oben erwähnt, das zweite, separate Gehäuse relativ geringe Außenabmessungen aufweisen kann und daher im Hinblick auf den Implantationsort im menschlichen Körper weitaus geringeren Beschränkungen unterliegt, als das erste, die Spannungsquelle und den Kondensator beinhaltende Kapselgehäuse, ist entsprechend einer vorteilhaften Weiterbildung der erfindungsgemäßen Defibrillatoranordnung vorgesehen, daß das separate Gehäuse eine zumindest bereichsweise elektrisch leitende und als eine der Elektroden ausgebildete Oberfläche aufweist. Das zweite, separate Gehäuse wird dann am oder in der Nähe des Herzens implantiert und übernimmt dort zusätzlich die Funktion einer Elektrode.

Das Anwendungsspektrum der erfindungsgemäßen Defibrillatoranordnung wird in vorteilhafter Weise durch eine Auswahl weiterer separater Gehäuse mit unterschiedlichen Werten für die jeweils darin angeordnete Induktivität bzw. das zumindest eine passive elektrische Bauelement erhöht.

Zur Erläuterung der Erfindung wird in folgenden auf die Figuren der Zeichnung Bezug genommen; im einzelnen zeigen
FIG 1 ein erstes Ausführungsbeispiel der erfindungsgemäßen Defibrillatoranordnung mit einem ersten und einem zweiten Gehäuse.
FIG 2 eine weiteres Ausführungsbeispiel mit einer Stromteilerschaltung in dem zweiten Gehäuse und
FIG 3 ein drittes Ausführungsbeispiel, bei dem das zweite Gehäuse eine Elektrode bildet.

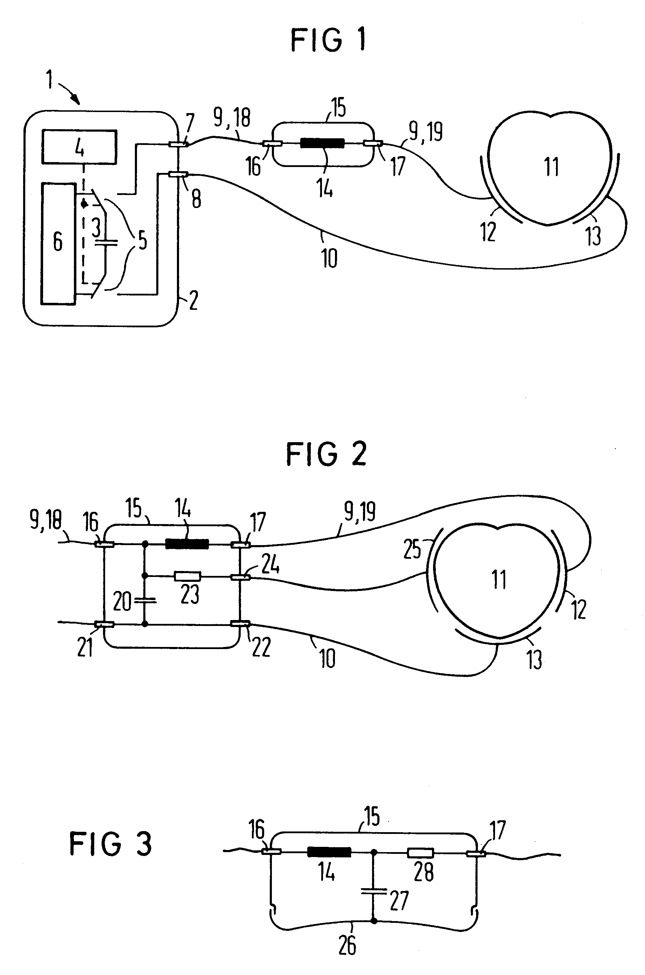

FIG 1 zeigt einen zur Abgabe monophasischer Defibrillationsimpulse ausgebildeten herkömmlichen Defibrillator 1, bei dem in einem implantierbaren Kapselgehäuse 2 ein Kondensator 3 angeordnet ist, der über eine von einer Steuereinrichtung 4 steuerbare Schalteranordnung 5 entweder an eine Spannungsquelle 6 oder an zwei von außen zugängliche Elektrodenanschlüsse 7 und 8 schaltbar ist. Die Elektrodenanschlüsse 7 und 8 sind über Elektrodenzuleitungen 9 und 10 mit zwei an einem Herzen 11 plazierten Elektroden 12 und 13 verbunden. In den Verlauf der mit 9 bezeichneten Elektrodenzuleitung ist eine Induktivität 14 eingefügt, die in einem zweiten, separaten Gehäuse 15 angeordnet ist und an Anschlüssen 16 und 17 über einen ersten Leitungsabschnitt 18 der Elektrodenzuleitung 9 mit dem Ausgangsanschluß 7 des Defibrillators 1 und über einen zweiten Leitungsabschnitt 19 der Elektrodenzuleitung 9 mit der mit 12 bezeichneten Elektrode verbunden ist. Die Gehäuse 2 und 15 bestehen jeweils aus einem gewebeverträglichem Material, beispielsweise Titan wobei die Anschlüsse 7,8,16 und 17 in Form von Isolationsdurchführungen durch das Metallgehäuse ausgebildet sind. Das separate Gehäuse 15 kann aber auch aus einem Kunststoff, beispielsweise Silikon bestehen, um es einfacher in die Elektrodenzuleitung 9 integrieren zu können oder um zur Erleichterung der Implantierbarheit unterschiedliche Gehäuseformen ausbilden zu können.

Solange der Kondensator 3 über die Schalteranordnung 5 mit der Spannungsquelle 6 verbunden ist, wird er auf eine vorgegebene Ladespannung aufgeladen. Zur Abgabe eines elektrischen Defibrillationsimpulses an das Herz 11 wird der Kondensator 3 mittels der Schalteranordnung 5 kurzzeitig über die Induktivität 14 mit den Elektroden 12 und 13 verbunden, wobei ein biphasischer Stromimpuls in Form einer stark gedämpften Schwingung erzeugt wird. Die Form des Stromimpulses ist durch die Werte für die Kapazität des Kondensators 3, die Induktivität 14 und die Impedanz des Herzgewebes zwischen den Elektroden 12 und 13 bestimmt. Da sich der Wert für die Impedanz des Herzgewebes zwischen den Elektroden 12 und 13 in Abhängigkeit von deren Größe und Anordnung am Herzen 11 ändert, kann der dem Herzen 11 zugeführte Defibrillationsimpuls je nach Anordnung und Größe der Elektroden 12 und 13 von einer als im Hinblick auf die Effektivität der Defibrillations als optimal geltenden Impulsform abweichen. Dieser Abweichung läßt sich in einfacher Weise durch die Wahl eines geeigneten Wertes für die Induktivität 14 entgegenwirken, wobei lediglich das zweite separate Gehäuse 15 mit der entsprechenden Induktivität 14 entweder erstmalig zusammen mit dem Defibrillator 1 oder als Austausch gegen ein bereits vorhandenes separates Gehäuse in dem Patienten implantiert werden muß. Da das separate Gehäuse 15 relativ kleine Abmessungen aufweisen kann, kann es an einer für einen Austausch günstigen Stelle im Patientenkörper implantiert werden.

Zur weiteren Optimierung des Defibrillationsimpulses kann bei dem in FIG 1 gezeigten Ausführungsbeispiel in dem zweiten separaten Gehäuse 15 zusätzlich zu der Induktivität 14 ein weiteres, hier nicht gezeigtes passives elektrisches Bauelement in Form eines Widerstandes oder eines Kondensators vorgesehen werden, das entweder in der Reihe mit der Induktivität 14 oder parallel zu dieser zwischen den Anschlüssen 16 und 17 angeordnet ist; ein entsprechendes Ausführungsbeispiel zeigt die weiter unten erläuterte FIG 3.

Wie FIG 2 zeigt, kann das weitere passive elektrische Bauelement 20 (hier eine Kapazität) auch zwischen den verschiedenen Elektrodenzuleitungen 9 und 10 angeordnet sein, wozu das zweite, separate Gehäuse 15 mit weiteren Anschlüssen 21 und 22 für die mit 10 bezeichnete Elektrodenzuleitung versehen ist. Außerdem enthält das in FIG 2 gezeigte separate Gehäuse 15 ein zusätzliches passives elektrisches Bauelement 23, hier in Form eines Widerstandes, der an einer Anschlußseite mit dem über den Leitungsabschnitt 18 an dem hier nicht gezeigten Defibrillator 1 angeschlossenen Anschluß 16 verbunden ist und an seiner anderen Anschlußseite mit einem zusätzlichen Anschluß 24 für eine weitere am Herzen 11 plazierte Elektrode 25 verbunden ist. Auf diese Weise wird erreicht, daß sich bei der Abgabe des Defibrillationsimpulses die elektrische Stromdichte im Herzmuskel entsprechend der Anordnung der Elektroden 12,13 und 25 und in Abhängigkeit von den Werten für die Bauelemente 14,20 und 23 verteilt und dabei vorzugsweise die dicksten Zonen des Herzmuskels, die den Haupteil der Herzmuskelmasse bilden, durchdringt, um eine sichere Defibrillation zu erzielen.

FIG 3 zeigt schließlich ein Ausführungsbeispiel für das zweite, separate Gehäuse 15, bei dem ein Teilbereich 26 der Gehäuseoberfläche als Elektrode ausgebildet ist, die über einen Kondensator 27 mit dem Spannungsabgriff einer zwischen den Anschlüssen 16 und 17 angeordneten Reihenschaltung aus der Induktivität 14 und einem Widerstand 28 verbunden ist. Dabei wird die Anordnung des Gehäuses 15 mit dem als Elektrode ausgebildeten Teilbereich 26 der Gehäuseoberfläche am Herzen durch die geringen erforderlichen Außenabmessungen des Kapselgehäuses 15 begünstigt.

Die dargestellten Ausführungsbeispiele zeigen den Aufbau des separaten Gehäuses 15 nur in schematischer Darstellung. So kann die jeweilige Gehäuseform in Abhängigkeit von der als bevorzugt anzusehenden Implantationsstelle im Körper des Patienten ausgebildet sein.

Schließlich ist die gezeigte Auswahl und Anordnung von passiven Bauelementen 14, 20, 23, 27 und 28 lediglich als Beispiel für eine Vielzahl weiterer möglicher Ausführungsbeispiele zu sehen.

### Bezugszeichenliste

- 1: Defibrillator
- 2: Kapselgehäuse für 1
- 3: Kondensator
- 4: Steuereinrichtung
- 5: steuerbare Schalteranordnung
- 6: Spannungsquelle
- 7,8: Elektrodenanschlüsse
- 9,10: Elektrodenzuleitungen
- 11: Herz
- 12,13: Elektroden
- 14: Induktivität
- 15: zweites, separates Gehäuse
- 16,17: Anschlüsse von 15
- 18,19: Leitungsabschnitte von 9
- 20: weiteres passives Bauelement
- 21,22: weitere Anschlüsse von 15
- 23: zusätzliches passives Bauelement
- 24: zusätzlicher Anschluß von 15
- 25: weitere Elektrode
- 26: als Elektrode ausgebildeter Teilbereich der Gehäuseoberfläche von 15
- 27: Kondensator
- 28: Widerstand

## Patentansprüche

1. Implantierbare Defibrillatoranordnung mit einem in einem Kapselgehäuse (2) angeordneten Kondensator (3), der über eine steuerbare Schalteranordnung (5) zum Aufladen mit einer Spannungsquelle (6) und zur Abgabe eines elektrischen Defibrillationsimpuls über eine Induktivität (14) und Elektrodenzuleitungen (9,10) mit im Bereich des Herzens (11) angeordneten Elektroden (12,13) verbindbar ist, **dadurch gekennzeichnet**, daß die Induktivität (14) in einem zweiten, separaten Gehäuse (15) angeordnet ist, das mit Anschlüssen (16,17) zum Einfügen der Induktivität (14) in den Verlauf einer der Elektrodenzuleitungen (9) versehen ist.

2. Implantierbare Defibrillatoranordnung nach Anspruch 1, **dadurch gekennzeichnet**, daß in dem zweiten, separaten Gehäuse (15) zusätzlich zu der Induktivität (14) zumindest ein weiteres passives elektrisches Bauelement (28) zwischen den Anschlüssen (16,17) angeordnet ist.

3. Implantierbare Defibrillatoranordnung nach Anspruch 1 oder 2, **dadurch gekennzeichnet**, daß das separate Gehäuse (15) zumindest einen zusätzlichen Anschluß (24) für eine weitere Elektrode (25) aufweist und zumindest ein zusätzliches passives elektrisches Bauelement (23) enthält, welches mit der Induktivität (14) zu einer Stromteilerschaltung angeordnet ist, die den von dem Kondensator (3) abgegebenen Defibrillationsimpuls in unterschiedliche Teilströme für die Elektroden (12,13,25) aufteilt.

4. Implantierbare Defibrillatoranordnung nach einem der vorangehenden Ansprüche, **dadurch gekennzeichnet**, daß das separate Gehäuse (15) eine zumindest bereichsweise elektrisch leitende und als eine der Elektroden ausgebildete Oberfläche (26) aufweist.

5. Implantierbare Defibrillatoranordnung nach einem der vorangehenden Ansprüche, **gekennzeichnet durch** eine Auswahl weiterer separater Gehäuse mit unterschiedlichen Werten für die jeweils darin angeordnete Induktivität bzw. das zumindest eine passive elektrische Bauelement.

## Claims

1. Implantable defibrillator arrangement, having arranged in a capsule housing (2) a capacitor (3), which, for charging, can be connected by way of a controllable switching arrangement (5) to a voltage source (6), and, for the emission of an electric defibrillation pulse, can be connected by way of said controllable switching arrangement (5), via an inductor (14) and electrode supply lines (9, 10), to electrodes (12, 13) arranged in the region of the heart (11), characterised in that the inductor (14) is arranged in a second, separate housing (15), which is provided with terminals (16, 17) for the insertion of the inductor (14) into the course of one of the electrode supply lines (9).

2. Implantable defibrillator arrangement according to claim 1, characterised in that arranged in the second, separate housing (15), in addition to the inductor (14), there is at least one further passive electric component (28) between the terminals (16, 17).

3. Implantable defibrillator arrangement according to claim 1 or 2, characterised in that the separate housing (15) has at least one additional terminal (24) for a further electrode (25) and contains at least one additional passive electric component (23), which is arranged with the inductor (14) to form a current dividing circuit arrangement, which divides the defibrillation pulse emitted by the capacitor (3) into different component currents for the electrodes (12, 13, 25).

4. Implantable defibrillator arrangement according to one of the preceding claims, characterised in that the separate housing (15) has a surface (26) which is electroconductive at least in areas and is formed as one of the electrodes.

5. Implantable defibrillator arrangement according to one of the preceding claims, characterised by a choice of further separate housings with different values for the respective inductor arranged therein and the at least one passive electric component.

## Revendications

1. Défibrillateur implantable comportant un condensateur (3) disposé dans un boîtier d'encapsulation (2) et qui peut être relié, par l'intermédiaire d'un commutateur commandable (5), en vue de la charge, avec une source de tension (6) et en vue de l'émision d'une impulsion électrique de défibrillation, par l'intermédiaire d'une inductance (14) et de conducteurs (9,10) d'alimentation d'électrodes, à des électrodes (12,13) disposées au voisinage du coeur (11), caractérisé par le fait que l'inductance (14) est disposée dans un second boîtier séparé (15), qui est pourvu de bornes (16,17) pour l'insertion de l'inductance (14) dans le trajet de l'un des conducteurs (9) d'alimentation d'électrodes.

2. Défibrillateur implantable suivant la revendication 1, caractérisé par le fait qu'en plus de l'inductance (14), au moins un autre composant électrique passif (28) est disposé entre les bornes (16,17), dans le second boîtier séparé (15).

3. Défibrillateur implantable suivant la revendication 1 ou 2, caractérisé par le fait que le boîtier séparé (15) possède au moins une borne supplémentaire (24) pour une autre électrode (25) et contient au moins un composant électrique passif supplémentaire (23), qui est disposé de manière à former avec l'inductance (14) un circuit diviseur de courant, qui divise l'impulsion de défibrillation, délivrée par le condensateur (3), en des courants partiels différents pour les électrodes (12,13,25).

4. Défibrillateur implantable suivant l'une des revendications précédentes, caractérisé par le fait que le boîtier séparé (15) possède une surface (26) électriquement conductrice au moins par zones et agencée pour former l'une des électrodes.

5. Défibrillateur implantable suivant l'une des revendications précédentes, caractérisé par un choix d'autres boîtiers séparés possédant des valeurs différentes pour l'inductance disposée respectivement dans ces boîtiers, ou pour au moins un composant électrique passif.
